# EUROPEAN PATENT APPLICATION

(11) **EP 4 302 832 A1**
(43) Date of publication of application: **10.01.2024**
(21) Application number: 22183704.0
(22) Date of filing: 07.07.2022
(51) Int. Cl.: A61P 35/00, A61K 9/20, A61K 9/24, A61K 31/519

(54) **PALBOCICLIB FORMULATION CONTAINING GLUCONO DELTA LACTONE**

(71) Applicant: Lotus Pharmaceutical Co., Ltd., 11046 Taipei City (TW)
(72) Inventor: Mehta, Gaurav Bhupendrabhai, Nantou City (TW); Gattani, Yogesh Sevaramji, Nantou City (TW); Gupta, Vijender, Nantou City (TW); Chawla, Manish, Nantou City (TW)
(74) Representative: Harber IP s.r.o.

(57) **Abstract**

The present invention relates to a pharmaceutical solid composition, containing palbociclib or a pharmaceutically acceptable salt thereof; gluconic acid and/or derivative thereof; and optionally one or more further water-soluble acids.

The composition shows improved dissolution properties, compared to prior art compositions.

## Description

### Field Of Art

The present invention relates to a pharmaceutical composition containing 6-acetyl-8-cyclopentyl-5-methyl-2-(5-piperazin-1-yl-pyridin-2-ylamino)-8H-pyrido[2,3-d]-pyrimidin-7-one (Palbociclib) with desirable pharmacokinetic characteristics and exhibiting favourable storage stability and dissolution properties.

### Background Art

Palbociclib is a potent and selective inhibitor of CDK4 and CDK6, having the structure:

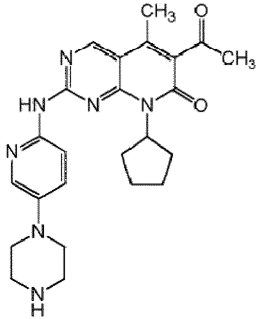

Palbociclib is a dibasic compound having two basic groups with pKa of approximately 7.3 (the secondary piperazine nitrogen) and 4.1 (the pyridine nitrogen). The solubility of palbociclib free base is pH dependent. Palbociclib is water soluble at low pH (2.1-4.5), while the solubility dramatically decreases as pH rises above 4.5. Palbociclib has poor water solubility (9 mg/mL) at pH 7.9. Simultaneous administration of agents which increase gastric pH can alter the solubility and absorption of palbociclib free base formulations.

The absorption and bioavailability of a therapeutic agent can be affected by numerous factors when administered orally. These factors may include the use of certain medications, such as proton pump inhibitors (PPIs) or H2 receptor antagonists, as well as certain medical conditions. Compounds having pH-dependent solubility, particularly basic compounds, may exhibit undesirable pharmacokinetic properties, such as poor absorption and/or reduced bioavailability, which may result in significant inter-patient and intra-patient variability.

Palbociclib-free base and pharmaceutically acceptable salts thereof are disclosed in WO2003062236A. Crystalline form of Palbociclib free base having a specific surface area of ≤ 2 m²/g is described in WO2014128588A. Immediate-release capsules containing Palbociclib free base in crystalline anhydrous Form A are approved under the brand name Ibrance^{®} (Pfizer) in EU and US. The marketed Palbociclib capsules comprise 75, 100, or 150 mg of Palbociclib in crystalline anhydrous Form A.

WO2005005426A describes an isethionate salt of Palbociclib.

Palbociclib is approved or was successfully tested for the treatment of ER+ breast cancer and HR+ breast cancer.

WO2016193860A discloses a solid dosage form comprising Palbociclib, a water-soluble acid selected from the group consisting of succinic acid, malic acid and tartaric acid, and a pharmaceutically acceptable carrier. The described solid dosage forms show excellent storage stability and provide substantially pH-independent delivery of Palbociclib with no significant food effects or adverse interactions with PPIs.

There still remains a need to provide improved dosage forms of palbociclib having improved dissolution and pharmacokinetic profiles, which also exhibit excellent storage stability.

### Detailed description of the Invention

We have surprisingly found that pharmaceutical solid compositions according to the present invention containing Palbociclib (free base and/or pharmaceutically acceptable salt) and gluconic acid and/or derivatives thereof, optionally in combination with other water-soluble acids, demonstrate excellent storage stability, improved dissolution profile and provide substantially pH-independent delivery of Palbociclib with no significant food effect.

The pH of the GI tract may vary based on whether a patient or subject is in a fed or fasted state. In general, the gastric residence time of a drug is longer in the presence of food than in the fasted state. If the bioavailability of a drug is significantly affected by the presence or absence of food in the GI tract, the drug is said to exhibit a "food effect". The rate of gastric emptying may also influence the concentration of drugs in solution available for absorption at different sites along the GI tract.

The present invention thus provides a pharmaceutical solid composition containing
- Palbociclib or a pharmaceutically acceptable salt thereof;
- gluconic acid and/or derivative thereof; and
- optionally one or more further water-soluble acids.

Palbociclib is preferably present in the composition in the form of free base. More preferably, palbociclib is present in crystalline form. Yet more preferably, palbociclib free base crystalline form has a specific surface area (measured as BET) at least 2.3 m²/g.

Pharmaceutically acceptable salts of palbociclib may include palbociclib isethionate salt.

Derivatives of gluconic acid are preferably selected from glucono-delta lactone (GDL), sodium gluconate, calcium gluconate, potassium gluconate. Most preferably, the gluconic acid derivative is glucono-delta lactone.

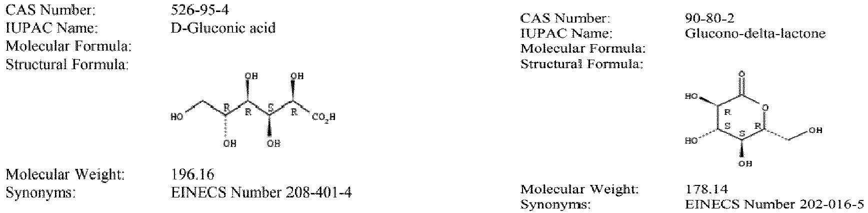

Glucono-delta-lactone (GDL), the inner ester of gluconic acid, is formed by the removal of water. Commercially it is produced by an anaerobic fermentation process converting a carbohydrate source into gluconic acid and glucono-delta-lactone. After fermentation, the gluconic acid is separated from GDL by crystallization. Glucono-delta-lactone (GDL) is a convenient substitute for the free acid, especially where, due to the reversibility between the acid and its lactone form, a gradual change in the pH is preferred.

The further water-soluble acids preferably include citric acid, succinic acid, fumaric acid, tartaric acid, malic acid, lactic acid, glutamic acid, L-glutamic acid, glutamic acid hydrochloride, Hydrochloric acid. The water-soluble acid is suitable to maintain acidic pH of the pharmaceutical composition.

In some embodiments, the composition of the invention may contain an intragranular phase containing palbociclib or a pharmaceutically acceptable salt thereof, and an extragranular phase containing the gluconic acid and/or derivative thereof. The further water-soluble acids, if present, are typically present in the same phase as the gluconic acid and/or derivative thereof.

In some embodiments, the composition of the invention may contain at least two layers, one layer containing palbociclib or a pharmaceutically acceptable salt thereof, and a different layer containing the gluconic acid and/or derivative thereof. The further water-soluble acids, if present, are typically present in the same layer as the gluconic acid and/or derivative thereof.

In some embodiments, the amount of palbociclib or a pharmaceutically acceptable salt thereof in the solid composition of the invention is within the range of 10 to 50 wt. %, preferably 15 to 42 wt. %, more preferably 17 to 23 wt. % or 38 to 42 wt. %.

In some embodiments, the amount of gluconic acid and/or derivative thereof in the solid composition of the invention is within the range of 0.5 to 25 wt.%, preferably 4 to 15 wt. %, more preferably 5 to 7 wt. % or 8 to 15 wt. %.

In some embodiments, the amount of palbociclib or a pharmaceutically acceptable salt thereof in the solid composition of the invention is within the range of 10 to 50 wt. % and the amount of gluconic acid and/or derivative thereof in the composition of the invention is within the range of 0.5 to 25 wt.%, preferably 4 to 15 wt. %.

In some embodiments, the amount of palbociclib or a pharmaceutically acceptable salt thereof in the solid composition of the invention is within the range of 15 to 42 wt. % and the amount of gluconic acid and/or derivative thereof in the composition of the invention is within the range of 0.5 to 25 wt.%, preferably 4 to 15 wt. %.

In some embodiments, the amount of palbociclib or a pharmaceutically acceptable salt thereof in the solid composition of the invention is within the range of 17 to 23 wt. % and the amount of gluconic acid and/or derivative thereof in the composition of the invention is within the range of 5 to 7 wt.%.

In some embodiments, the amount of palbociclib or a pharmaceutically acceptable salt thereof in the solid composition of the invention is within the range of 38 to 42 wt. % and the amount of gluconic acid and/or derivative thereof in the composition of the invention is within the range of 8 to 15 wt.%.

The solid composition of the invention further contains at least one pharmaceutically acceptable excipient, preferably selected from disintegrants, binders, diluents, lubricants, and glidants.

Disintegrants may be added to the pharmaceutical granulate composition to promote the breakup of the tablet or capsule into smaller fragments in an aqueous environment, thereby increasing the available surface area and promoting a more rapid release of the active pharmaceutical ingredient. Suitable examples of disintegrants to be used according to the present invention include crospovidone, sodium starch glycolate, croscarmellose sodium, and mixtures of any of the foregoing. Preferred disintegrant is sodium starch glycolate or crospovidone. Disintegrants can be added as intragranular or extragranular excipients. Disintegration occurs by the rapid uptake of water followed by rapid and enormous swelling. When added as an intragranular excipient it helps to break the granules whereas when added as an extragranular excipient it helps the tablet or capsule to disintegrate. In a preferred embodiment of the present invention, the disintegrant is added extragranularly and intragranularly.

The pharmaceutical formulation further contains a binder which is selected from the group of povidone, co-povidone, hydroxypropyl methylcellulose, hydroxy propyl cellulose, and sodium carboxyl methylcellulose.

Diluents are fillers that are used to increase the bulk volume of the pharmaceutical granulate composition. Generally, by combining a diluent with the active pharmaceutical ingredient, the final product is given adequate weight and size to assist in production and handling. Suitable examples of diluents to be used according to the present invention include starch, pregelatinized starch, microcrystalline cellulose, calcium phosphate, lactose, sorbitol, mannitol, and sucrose. According to the preferred embodiment of the invention microcrystalline cellulose and/or lactose are used as diluents.

Lubricants are preferably selected from the group containing magnesium stearate, calcium stearate, stearic acid, glyceryl behenate, hydrogenated castor oil, glycerine fumarate.

The pharmaceutical composition according to the invention is prepared by using different granulation methods, preferably selected from dry or wet granulation. Dry granulation includes dry mixing, slug-de-slug and roller compaction processes. Wet granulation includes aqueous granulation and non-aqueous granulation. Other wet granulation technologies like Extrusion - Spherization, Spray drying, Supercritical fluid, Fluid Bed Granulation, Hotmelt Granulation, freeze granulation, Foam Granulation etc. are also suitable. Preferably, dry granulation or direct compression is used, or granulation followed by filling of capsules.

The composition of the invention is preferably formulated as a tablet or a coated tablet or a capsule. The coated tablet may be a film-coated tablet. The tablet may be a bi-layer or a multi-layer tablet.

The solid composition of the invention is suitable for use in the treatment of cancer, more specifically in the treatment of ER+ or HR+ breast cancer.

### Examples of carrying out the Invention

### Examples 1-3

A solid pharmaceutical composition comprising Palbociclib and a water-soluble acid Glucono-delta-lactone (GDL) prepared by using direct compression process.

Palbociclib (free base, preferably with specific surface area NLT 2.3 m²/g specified by BET method), Microcrystalline cellulose (trade name: MCC, FMC), Glucono-delta-lactone (GDL) (trade name: GDL, Roquette), Crospovidone (trade name: VIVAPHARM ^{®} PVPP, JRS pharma), Colloidal silicon dioxide (trade name: Aerosil 200 Pharma, Evonik), Magnesium stearate (From Peter Greven) according to the formulation proportions in Table 1 was mixed using a diffusion mixer. The blend was compressed into tablet, and the tablet was further coated by Opadry purple. The drug content in the composition is about 41 % w/w calculated based on the total weight of the coated tablet.

**Table: 1**

| | **Strength: 125 mg** | **Example 1** | | **Example 2** | | **Example 3** | |
|---|---|---|---|---|---|---|---|
| **Sr No.** | **Ingredients** | **mg/Tablet** | **%w/w** | **mg/Tablet** | **%w/w** | **mg/Tablet** | **%w/w** |
| **1** | Palbociclib | 125.000 | 41.25 | 125.000 | 41.25 | 125.000 | 41.25 |
| **2** | Microcrystalline cellulose | 65.000 | 21.45 | 65.000 | 21.45 | 65.000 | 21.45 |
| **3** | Colloidal silicon dioxide | 5.500 | 1.82 | 5.500 | 1.82 | 5.500 | 1.82 |
| **4** | Crospovidone | 18.750 | 6.19 | 18.750 | 6.19 | 18.750 | 6.19 |
| **5** | Magnesium stearate | 3.000 | 0.99 | 3.000 | 0.99 | 3.000 | 0.99 |
| **6** | Crospovidone | 18.750 | 6.19 | 18.750 | 6.19 | 18.750 | 6.19 |
| **7** | Glucono-delta-lactone (GDL) | 35.000 | 11.55 | 45.000 | 14.85 | 25.000 | 8.25 |
| **8** | Microcrystalline cellulose | 20.000 | 6.60 | 10.000 | 3.30 | 30.000 | 9.90 |
| **9** | Magnesium stearate | 3.000 | 0.99 | 3.000 | 0.99 | 3.000 | 0.99 |
| | Total uncoated tablet weight | 294.000 | 97.03 | 294.000 | 97.03 | 294.000 | 97.03 |
| **10** | Coating -Opadry Purple | 9.000 | 2.97 | 9.000 | 2.97 | 9.000 | 2.97 |
| | Total weight of tablets | 303.000 | 100.00 | 303.000 | 100.00 | 303.000 | 100.00 |

### Examples 4-6

A solid composition in the form of a tablet comprising Palbociclib and a water-soluble acid Glucono-delta-lactone (GDL) prepared by using dry granulation process.

The solid composition contains Palbociclib (Free base, preferably with Specific surface area NLT 2.3 m²/g specified by BET method), Microcrystalline cellulose (trade name: MCC, FMC), Glucono-delta-lactone (GDL) (trade name: GDL, Roquette), crospovidone (trade name: VIVAPHARM ^{®} PVPP, JRS pharma), colloidal silicon dioxide (trade name: Aerosil 200 Pharma, Evonik), Magnesium stearate (From Peter Greven) according to Table 2.

The intragranular part 1-6 of the formulation in Table 2 was thoroughly mixed using a diffusion mixer, granulated by roller compaction, and followed by milling through co-mill 1.2 mm. The granules were further lubricated, and the extra granular part of excipients was added. The granules with the extra granular excipients were compressed into tablet, and the tablet was further coated by Opadry purple. The drug content is about 19 % w/w calculated based on the total weight of the coated tablet.

**Table 2**

| | **Strength: 125 mg** | **Example 4** | | **Example 5** | | **Example 6** | |
|---|---|---|---|---|---|---|---|
| **Sr No.** | **Ingredients** | **mg/Tablet** | **%w/w** | **mg/Tablet** | **%w/w** | **mg/Tablet** | **%w/w** |
| **Intragranular material** | | | | | | | |
| **1** | Palbociclib | 125.000 | 19.23 | 125.000 | 19.23 | 125.000 | 19.23 |
| **2** | Microcrystalline cellulose | 240.000 | 36.92 | 240.000 | 36.92 | 240.000 | 36.92 |
| **3** | Colloidal silicon dioxide | 13.000 | 2.00 | 13.000 | 2.00 | 13.000 | 2.00 |
| **4** | Crospovidone | 18.750 | 2.88 | 18.750 | 2.88 | 18.750 | 2.88 |
| **5** | Glucono-delta-lactone (GDL) | -- | -- | -- | -- | 3.000 | 0.46 |
| **6** | Magnesium stearate | 13.000 | 2.00 | 13.000 | 2.00 | 13.000 | 2.00 |
| | **Weight in Intragranular** | **409.750** | **63.04** | **409.750** | **63.04** | **412.750** | **63.50** |

| **Extragranular material** | | | | | | | |
|---|---|---|---|---|---|---|---|
| 7 | Crospovidone | 18.750 | 2.88 | 18.750 | 2.88 | 18.750 | 2.88 |
| **8** | Glucono-delta-lactone (GDL) | 35.000 | 5.38 | 45.000 | 6.92 | - | - |
| **9** | Microcrystalline cellulose | 154.500 | 23.77 | 144.500 | 22.23 | 186.500 | 28.69 |
| **10** | Magnesium stearate | 13.000 | 2.00 | 13.000 | 2.00 | 13.000 | 2.00 |
| | Total uncoated tablet weight | 631.000 | 97.08 | 631.000 | 97.08 | 631.000 | 97.08 |
| **11** | Coating -Opadry Purple | 19.000 | 2.92 | 19.000 | 2.92 | 19.000 | 2.92 |
| | Total weight of tablets | 650.000 | 100.00 | 650.000 | 100.00 | 650.000 | 100.00 |

### Examples 7-8

A solid composition in form of bi-layer tablet comprising Palbociclib and water-soluble acid-Glucono-delta-lactone (GDL) and/or L-glutamic acid hydrochloride prepared by using dry granulation process.

The solid composition contains Palbociclib (Freebase, Specific surface area NLT 2.3 m²/g by BET method), Microcrystalline cellulose (trade name: MCC, FMC), ( from Merck), Crospovidone (trade name: VIVAPHARM ^{®} PVPP, JRS pharma), colloidal silicon dioxide (trade name: Aerosil 200 Pharma, Evonik), Magnesium stearate (From Peter Greven), Glucono-delta-lactone (GDL) (trade name: GDL, Roquette) or L-glutamic acid hydrochloride (From Merck) according to the formulation proportions in Table 3.

The bi-layer tablets were prepared by using dry granulation process.

The components 1-4 of the first layer according to table 3 were thoroughly mixed using a diffusion mixer, granulated by roller compaction, followed by milling through co-mill 1.2 mm and the granules were further lubricated.

The components of the second layer Glucono-delta-lactone (GDL) or L-glutamic acid hydrochloride, Microcrystalline cellulose, Crospovidone, colloidal silicon dioxide, Magnesium stearate were mixed in a diffusion mixer.

The granules of the first layer (active layer) and the blend of the second layer (acidifier layer) were further compressed using suitable tooling of the bi-layer tablet with total weight about 631 mg and the bi-layer tablet was further coated by using Opadry purple. The drug content is about 19 % w/w of the total weight of the film-coated tablet.

**Table 3**

| | **Strength: 125 mg** | **Example 7** | | **Example 8** | |
|---|---|---|---|---|---|
| **Sr No.** | **Ingredients** | **mg/Tablet** | **%w/w** | **mg/Tablet** | **%w/w** |
| **layer one** | | | | | |
| **1** | Palbociclib | 125.000 | 19.23 | 125.000 | 19.23 |
| **2** | Microcrystalline cellulose | 240.250 | 36.96 | 240.250 | 36.96 |
| **3** | Colloidal silicon dioxide | 13.000 | 2.00 | 13.000 | 2.00 |
| **4** | Crospovidone | 18.750 | 2.88 | 18.750 | 2.88 |
| **5** | Magnesium stearate | 13.000 | 2.00 | 13.000 | 2.00 |
| | Total weight of layer one | 410.000 | 63.08 | 410.000 | 63.08 |

| **Layer two** | | | | | |
|---|---|---|---|---|---|
| **6** | Crospovidone | 18.750 | 2.88 | 18.750 | 2.88 |
| **7** | Glucono-delta-lactone (GDL) | 35.000 | 5.38 | 35.000 | 5.38 |
| **8** | L-glutamic acid hydrochloride | -- | -- | 30.000 | 4.62 |
| **9** | Microcrystalline cellulose | 154.250 | 23.73 | 124.250 | 19.12 |
| **10** | Magnesium stearate | 13.000 | 2.00 | 13.000 | 2.00 |
| | Total weight of layer two | 221.000 | 34.00 | 221.000 | 34.00 |
| **11** | Coating -Opadry Purple | 19.000 | 2.92 | 19.000 | 2.92 |
| | Total weight of film coated tablets | 650.000 | 100.00 | 650.000 | 100.00 |

### Examples 9-13

A solid formulation comprising Palbociclib and a mixture of water-soluble acid Glucono-delta-lactone (GDL) and succinic acid, malic acid, lactic acid, citric acid and tartaric acid prepared by dry granulation process.

The solid composition contains Palbociclib (Free base, preferably with Specific surface area NLT 2.3 m²/g specified by BET method), Microcrystalline cellulose (trade name: MCC, FMC), Glucono-delta-lactone (GDL) (trade name: GDL, Roquette), Citric acid (From Finar India), DL-Malic acid (From Finar India), Succinic acid (From Finar India), Tartaric acid (From Finar India), crospovidone (trade name: VIVAPHARM ^{®} PVPP, JRS pharma), colloidal silicon dioxide (trade name: Aerosil 200 Pharma, Evonik), Magnesium stearate (From Peter Greven) according to the formulation proportions in Table 4.

The intragranular part of the composition according to Table 4 was thoroughly mixed using a diffusion mixer, granulated by roller compaction, followed by milling through co-mill 1.2 mm and the final granules were further lubricated. The granules were mixed with the extra granular excipients according to Table 4 and compressed into tablet, and the tablet was further coated using Opadry purple. The drug content is about 19 % w/w of the total weight of the coated tablet.

**Table 4**

| | **Strength: 125 mg** | **Example 9** | | **Example 10** | | **Example 11** | | **Example 12** | | **Example 13** | |
|---|---|---|---|---|---|---|---|---|---|---|---|
| Sr No. | **Ingredients** | **mg/Tablet** | **%w/w** | **mg/Tablet** | **%w/w** | **mg/Tablet** | **%w/w** | **mg/Tablet** | **%w/w** | **mg/Tablet** | **%w/w** |
| **Intragranular material** | | | | | | | | | | | |
| **1** | Palbociclib | 125.000 | 19.23 | 125.000 | 19.23 | 125.000 | 19.23 | 125.000 | 19.23 | 125.000 | 19.23 |
| **2** | Microcrystalline cellulose | 240.000 | 36.92 | 240.000 | 36.92 | 240.000 | 36.92 | 240.000 | 36.92 | 240.000 | 36.92 |
| **3** | Colloidal silicon dioxide | 13.000 | 2.00 | 13.000 | 2.00 | 13.000 | 2.00 | 13.000 | 2.00 | 13.000 | 2.00 |
| **4** | Crospovidone | 18.750 | 2.88 | 18.750 | 2.88 | 18.750 | 2.88 | 18.750 | 2.88 | 18.750 | 2.88 |
| **5** | Magnesium stearate | 13.000 | 2.00 | 13.000 | 2.00 | 13.000 | 2.00 | 13.000 | 2.00 | 13.000 | 2.00 |
| | Weight at intragranular | 409.750 | 63.04 | 409.750 | 63.04 | 409.750 | 63.04 | 409.750 | 63.04 | 409.750 | 63.04 |

| **Extragranular material** | | | | | | | | | | | |
|---|---|---|---|---|---|---|---|---|---|---|---|
| **6** | Crospovidone | 18.750 | 2.88 | 18.750 | 2.88 | 18.750 | 2.88 | 18.750 | 2.88 | 18.750 | 2.88 |
| **7** | Glucono-delta -lactone (GDL) | 35.000 | 5.38 | 35.000 | 5.38 | 35.000 | 5.38 | 35.000 | 5.38 | 35.000 | 5.38 |
| **8** | Succinic acid | -- | -- | 16.000 | 2.46 | -- | -- | -- | -- | -- | -- |
| **9** | Citric acid | -- | -- | -- | -- | 70.000 | 10.77 | -- | -- | -- | -- |
| **10** | Malic acid | -- | -- | -- | -- | -- | -- | 16.000 | 2.46 | -- | -- |
| **8** | Tartaric acid | -- | -- | -- | -- | -- | -- | -- | -- | 16.000 | 2.46 |
| **9** | Lactic acid | 70.00 | 10.77 | -- | -- | -- | -- | -- | -- | -- | -- |
| **9** | Microcrystalline cellulose | 84.500 | 13.00 | 138.500 | 21.31 | 84.500 | 13.00 | 138.500 | 21.31 | 138.500 | 21.31 |
| **10** | Magnesium stearate | 13.000 | 2.00 | 13.000 | 2.00 | 13.000 | 2.00 | 13.000 | 2.00 | 13.000 | 2.00 |
| | Total uncoated tablet weight | 631.000 | 97.08 | 631.000 | 97.08 | 631.000 | 97.08 | 631.000 | 97.08 | 631.000 | 97.08 |
| **11** | Coating -Opadry Purple | 19.000 | 2.92 | 19.000 | 2.92 | 19.000 | 2.92 | 19.000 | 2.92 | 19.000 | 2.92 |
| | Total weight of film coated tablets | 650.000 | 100.00 | 650.000 | 100.00 | 650.000 | 100.00 | 650.000 | 100.00 | 650.000 | 100.00 |

### Examples 14-16

A solid formulation comprising Palbociclib and a mixture of water-soluble acid Gluconic acid or Hydrochloric acid prepared by dry granulation of the active layer and top spray granulation of the acidifier layer

The solid formulation contains 2 types of granules:
1. Palbociclib (Freebase, Specific surface area NLT 2.3 m²/g by BET method), Microcrystalline cellulose (trade name: MCC, FMC), Crospovidone (trade name: VIVAPHARM ^{®} PVPP, JRS pharma), colloidal silicon dioxide (trade name: Aerosil 200 Pharma, Evonik), Magnesium stearate (From Peter Greven) according to the formulation proportions in Table 6 was thoroughly mixed using a diffusion mixer, granulated by roller compaction, followed by milling through co-mill 1.2 mm and further lubricated.
2. Microcrystalline cellulose (trade name: MCC, FMC) was top spray granulated by Gluconic acid (From Roquette) ethanol solution - Table 5

The granules containing Palbociclib were mixed with the granules containing either MCC and Gluconic acid or MCC and Hydrochloric acid, mixed with Crospovidone and Magnesium stearate, compressed into tablet, and the tablet was further coated by Opadry purple.

**Table 5**

| | | **Example 14** | |
|---|---|---|---|
| **Sr No.** | **Ingredients** | **mg/Tablet** | **%w/w** |
| Acidifier - spray granulation | | | |
| 1 | Microcrystalline cellulose | 200.000 | 85.11 |
| 2 | Gluconic acid | 35.000 | 14.89 |
| total weight of granules | | 235.000 | 100.00 |

**Table 6**

| | **Strength: 125 mg** | **Example 15** | |
|---|---|---|---|
| **Sr No.** | **Ingredients** | **mg/Tablet** | **%w/w** |
| 1 | Palbociclib | 125.000 | 30.49 |
| 2 | Microcrystalline cellulose | 240.250 | 58.60 |
| 3 | Colloidal silicon dioxide | 13.000 | 3.17 |
| 4 | Crospovidone | 18.750 | 4.57 |
| 5 | Magnesium stearate | 13.000 | 3.17 |
| | Total weight of granules | 410.000 | 100.00 |

**Table 7**

| | **Strength: 125 mg** | **Example 16** | |
|---|---|---|---|
| **Sr No.** | **Ingredients** | **mg/Tablet** | **%w/w** |
| **1** | Granules of Example 15 | 410.000 | 59.08 |
| **2** | Granules of Example 14 | 235.000 | 33.86 |
| **3** | Crospovidone | 18.000 | 2.59 |
| **4** | Magnesium stearate | 12.000 | 1.73 |
| | Total uncoated tablet weight | 675.000 | 97.26 |
| **5** | Coating -Opary Purple | 19.000 | 2.74 |
| | Total weight of film coated tablets | 694.000 | 100.00 |

### Dissolution data:

The solid pharmaceutical compositions described in examples 4 and 10 were further evaluated for dissolution and compared with the reference products available on the market - Ibrance Capsules 125 mg, Ibrance film-coated tablets 125 mg from Pfizer Manufacturing Deutschland GmbH.

Dissolution conditions are the following:

| **Parameters** | **Dissolution Tester Conditions** |
|---|---|
| Medium | 0.1 N HCL, 900 mL |
| Apparatus | USP II (Paddle) |
| Temperature | 37.0 ± 0.5°C |
| Sampling Time | 10/15/20/30 mins |
| Sampling Volume | 10 mL |

Dissolution conditions at pH 4.5

| **Parameters** | **Dissolution Tester Conditions** |
|---|---|
| Medium | pH 4.5 Acetate Buffer, 900 mL |
| Apparatus | USP II (Paddle) |
| Temperature | 37.0 ± 0.5°C |
| Sampling Time | 10/15/20/30/45 mins |
| Sampling Volume | 10 mL |

Dissolution data of test formulations according to examples 4 and 10 show improved drug release compared to the marketed tablet formulation Ibrance film-coated tablets (containing succinic acid), and better dissolution than the marketed Ibrance Capsule formulation (not containing water-soluble acids).

### Stability testing

Stability testing was performed according to the ICH Harmonised Tripartite Guideline: Stability Testing of New Drug Substances and Products Q1A(R2), Step 4 version of 6 February 2003.

| Attribute | Specifications | Results at testing Intervals (Example 4) Accelerated Stability conditions - 40±2°C, 75±5%RH | | |
|---|---|---|---|---|
| | Time (Months) | 0 | 1 | 3 |
| Assay / Palbociclib | By HPLC method | 98.4% | 99.9% | 98.6% |
| Degradation product | Any unspecified impurity: | ND | ND | ND |
| | Total impurities: | 0.10% | 0.23% | 0.33% |

| | | | | |
|---|---|---|---|---|
| NP - not performed ND - not detected | | | | |

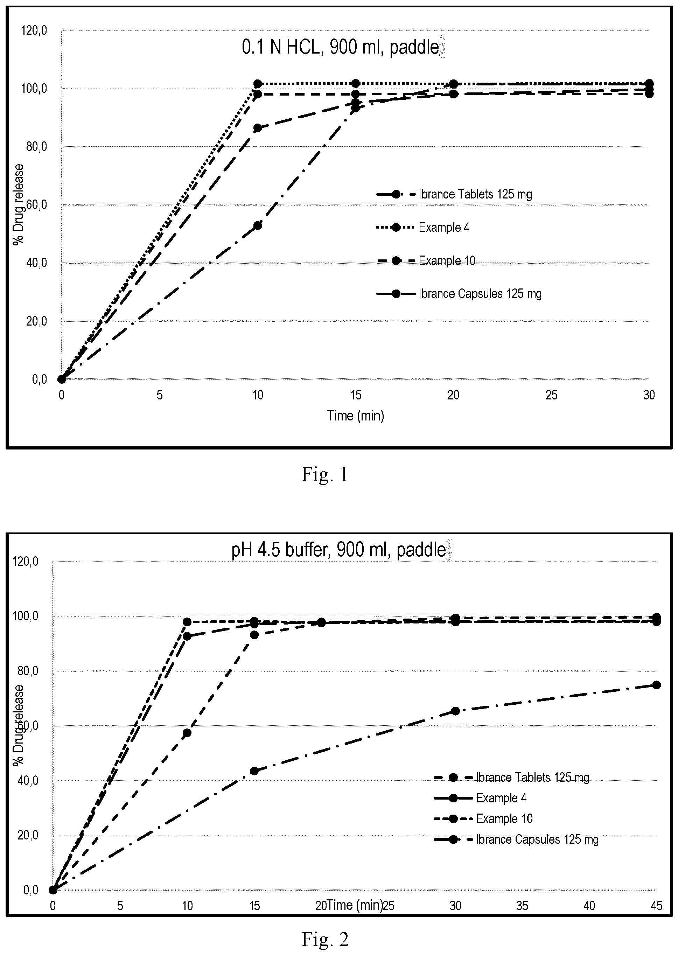

## Claims

1. Pharmaceutical solid composition, containing
- palbociclib or a pharmaceutically acceptable salt thereof;
- gluconic acid and/or derivative thereof; and
- optionally one or more further water-soluble acids.

2. The composition of claim 1, wherein palbociclib is present in the composition in the form of free base.

3. The composition of claim 1, wherein palbociclib is present in the composition as a free base crystalline form having a specific surface area (measured as BET) at least 2.3 m²/g.

4. The composition according to any one of the preceding claims, wherein the derivative of gluconic acid is selected from glucono-delta lactone, sodium gluconate, calcium gluconate, potassium gluconate.

5. The composition according to any one of the preceding claims, wherein the one or more further water-soluble acids is/are present and is/are selected from the group consisting of citric acid, succinic acid, fumaric acid, tartaric acid, malic acid, lactic acid, glutamic acid, L-glutamic acid, glutamic acid hydrochloride, hydrochloric acid.

6. The composition according to any one of the preceding claims, wherein the amount of palbociclib or a pharmaceutically acceptable salt thereof in the solid composition is within the range of 10 to 50 wt. %, preferably 15 to 42 wt. %.

7. The composition according to any one of the preceding claims, wherein the amount of gluconic acid and/or derivative thereof in the solid composition is within the range of 0.5 to 25 wt.%, preferably 4 to 15 wt. %.

8. The composition according to any one of the preceding claims, wherein the amount of palbociclib or a pharmaceutically acceptable salt thereof in the solid composition is within the range of 17 to 23 wt. % and the amount of gluconic acid and/or derivative thereof in the solid composition is within the range of 5 to 7 wt.%.

9. The composition according to any one of the preceding claims, wherein the amount of palbociclib or a pharmaceutically acceptable salt thereof in the solid composition is within the range of 38 to 42 wt. % and the amount of gluconic acid and/or derivative thereof in the solid composition is within the range of 8 to 15 wt.%.

10. The composition according to any one of the preceding claims, which further contains at least one pharmaceutically acceptable excipient, selected from disintegrants, binders, diluents, lubricants, and glidants.

11. The composition according to claim 10 which contains at least one disintegrant selected from sodium starch glycolate and crospovidone.

12. The composition according to any one of claims 1-11, which contains an intragranular phase containing palbociclib or a pharmaceutically acceptable salt thereof, and an extragranular phase containing the gluconic acid and/or derivative thereof.

13. The composition according to any one of claims 1-11, which contain at least two layers, one layer containing palbociclib or a pharmaceutically acceptable salt thereof, and a different layer containing the gluconic acid and/or derivative thereof.

14. The composition according to claim 12 or 13 which is selected from a tablet, a coated tablet and a capsule, more preferably selected from a film-coated tablet, a bi-layer tablet and a multi-layer tablet.

15. The solid composition according to any one of claims 1-14 for use in the treatment of cancer, preferably in the treatment of ER+ or HR+ breast cancer.
